# EUROPÄISCHE PATENTSCHRIFT

(11) **EP 0 325 259 B1**
(45) Veröffentlichungstag und Bekanntmachung des Hinweises auf die Patenterteilung: **20.10.1993**
(21) Anmeldenummer: 89100897.1
(22) Anmeldetag: 19.01.1989
(51) Int. Cl.: A61F 13/15

(54) **Wegwerfwindel**
Disposable napkin
Couche-culotte à jeter

(30) Priorität: 20.01.1988 JP 10332/88
(43) Veröffentlichungstag der Anmeldung: 26.07.1989
(73) Patentinhaber: UNI-CHARM CORPORATION, Kawanoe-shi Ehime-ken (JP)
(72) Erfinder: Yamamoto, Masamitsu, Kawanoe-shi Ehime-ken (JP); Igaue, Takamitsu, Kawanoe-shi Ehime-ken (JP); Sasaki, Tooru, Kawanoe-shi Ehime-ken (JP); Inoue, Kohji, Uma-gun Ehime-ken (JP)
(74) Vertreter: Sperling, Rüdiger, Dipl.-Ing.

(56) Entgegenhaltungen:
- EP-A- 0 109 126
- EP-A- 0 251 332
- FR-A- 2 388 515
- US-A- 3 452 753
- US-A- 3 825 006

## Beschreibung

Die vorliegende Erfindung betrifft eine Wegwerfwindel, insbesondere eine solche mit Seitenklappen, in denen elastische Teile eingearbeitet sind.

Es sind bereits unterschiedliche Arten von Wegwerfwindeln bekannt. Bezüglich solcher bekannter Wegwerfwindel, die Seitenklappen mit darin eingearbeiteten elastischen Teilen aufweisen, die einen angemessenen Druck um den Schenkel des Trägers ausüben, wurden zwei Typen von Wegwerfwindeln vorgeschlagen, nämlich eine solche, die einen flüssigkeitsabsorbierenden Kern aufweist, der zwischen einer flüssigkeitsdurchlässigen Oberlage und einer flüssigkeitsundurchlässigen rückwärtigen Lage sandwichartig eingelegt ist und deren Seitenklappen von Bereichen dieser beiden Lagen gebildet werden, die an den einander gegenüberliegenden Seitenrändern des Kerns seitlich abstehen, wobei in den Klappen elastische Teile angeordnet sind, um einen Druck um die Schenkel auszuüben, während der andere Typ als Teil der Seitenklappen eine dritte Lage Material verwendet, die separat auf der oberen und der rückwärtigen Lage angeordnet ist.

In einer derartigen bekannten Windel hat das elastische Teil üblicherweise eine Zugfestigkeit von 100 - 600 g, so daß das elastische Teil einen wirksamen Druck ausüben kann und eine gewünschte Abdichtung um den Schenkel bereitstellt. Vorzugsweise sollte das elastische Teil eine hohe prozentuale Kontraktion aufweisen, wenn es auf die Windel aufgebracht wird. Selbst bei üblichen Windeln wurde angestrebt, einen solche hohe prozentuale Kontraktion des elastischen Teils zu erzielen. Jedoch begrenzt eine relativ hohe Steifigkeit der Seitenklappen, in denen die elastischen Teile angeordnet sind, nachteiligerweise diese prozentuale Kontraktion. Um dieses Problem zu lösen kann versucht werden, daß das elastische Teil die gewünschte Kontraktion bereitstellt, d.h. man kann versuchen, ein elastisches Teil zu verwenden, das in seiner Elastizität hervorragend ist und gute Eigenschaften hinsichtlich der Dehnung aufweist. Es ist jedoch nicht leicht, ein derartiges elastisches Teil durch die heute zur Verfügung stehende Technik zu erhalten, und unvertretbare Kosten würden entstehen, selbst wenn ein solches elastisches Teil erhalten werden könnte.

In einem solchen Fall, bei dem in einer Wegwerfwindel die Seitenklappen zumindest teilweise durch die Oberlage gebildet aufweist, wird dieses Problem noch schwerwiegender, wenn als Oberlage eine solche mit relativ hoher Steifigkeit verwendet wird, um eine gewünschte Eigenschaft dieser Lage zu realisieren. Daraus folgt, daß Typus und Aufbau einer solchen Oberlage, die verwendet werden kann, begrenzt sind.

Aus der EP-A 0 251 332 ist eine Wegwerfwindel mit einer flüssigkeitsdurchlässigen oberen Lage, einer flüssigkeitsdurchlässigen rückwärtigen Lage und einem flüssigkeitsabsorbierenden Kern mit zwei Seitenklappen bekannt. Der flüssigkeitsabsorbierende Kern ist zwischen den genannten Lagen sandwichartig eingelegt, wobei sich die Seitenklappen an einander gegenüberliegenden Rändern des Kerns seitlich nach außen erstrecken. In den Seitenklappen sind elastische Teile angeordnet, die die Windel an den Beinöffnungen zusammenziehen. Jede der Seitenklappen ist außerhalb der oberen Lage angeordnet und weist eine dritte Lage auf, die zumindest einen Teil der Seitenklappe bildet.

Aus der EP-A 0 109 126 ist eine Wegwerfwindel bekannt, bei der die dritte Lage als Bestandteil der Seitenklappen eine zu den anderen Lagen unterschiedliche Steifigkeit aufweist. Diese bekannte Windel hat jedoch den Nachteil, daß die Steifigkeit der dritten Lage nicht so angepaßt ist, daß eine optimale Anschmiegung der Seitenklappen an den Schenkeln eines Trägers erzielt wird.

Der Erfindung liegt daher die Aufgabe zugrunde, eine Wegwerfwindel so zu schaffen, daß eine möglichts druckfreie, aber trotzdem dichte Umschließung der Schenkel des Trägers erzielt wird ohne den Typ und den Aufbau der Oberlage zuverändern.

Die Aufgabe wird erfindungsgemäß durch die Merkmale im kennzeichnenden Teil des Hauptanspruchs gelöst.

Um sicherzustellen, daß das elastische Teil die vorbestimmte prozentuale Kontraktion zeigt, selbst wenn dieses elastische Teil auch an der rückwärtigen Lage, die Teil dieser Seitenklappe bildet, anhaftet, weist die obere Lage eine Steifigkeit von 90 mm oder höher, gemessen mit einem Clark-Steifigkeitstestgerät gemäß des japanischen Industriestandards (JIS) (P8143) , auf; die rückwärtige Lage hat eine Steifigkeit geringer als 60 mm, gemessen mit diesem Tester, die dritte Lage weist eine Steifigkeit von 10 - 80 mm auf, gemessen mit diesem Tester; das elastische Teil besitzt eine Zugfestigkeit von 70 - 700 g, und die dritte Lage besitzt einen Wasserdruckwiderstand von 20 mm oder höher auf, gemessen mit einem Wasserdruckwiderstandstester gemäß der Japanischen Industriestandard (L1092) und besitzt luftdurchlässige Eigenschaften.

Erfindungsgemäß bildet die dritte Lage, deren Steifigkeit geringer ist als die der oberen Lage, einen Teil der Seitenklappe, wobei das elastische Teil zum Zwecke des Anpassens an den Schenkel des Trägers zumindest an der dritten Lage angeklebt ist. Ein derartiges Merkmal der Erfindung vergrößert vorteilhafterweise die Prozentuale Kontraktion, in anderen Worten ein Kontraktionsausmaß (d.h. in der Länge) bezüglich dem Fall, in welchem der genannte Teil der Seitenklappe aus einem Bereich der oberen Lage gebildet wird, der sich seitlich von dem Außenrand des Kerns wegerstreckt. Dies hat zur Folge, daß diese Seitenklappe, die aufgrund der Gegenwart des elastischen Teils in ihr eine Schrumpfung entwickelt, gut der Bewegung des Schenkels des Trägers folgt und sich diesem anpaßt und einen angemessenen Druck um den Schenkel herum ausübt, um eine sichere Abdichtung zu gewährleisten, wodurch mögliche Leckagen vermieden werden, die anderenfalls zwischen der Seitenklappe und dem Schenkel auftreten können. Des weiteren gestattet die vorliegende Erfindung, daß die obere Lage eine relativ hohe Steifigkeit, abhängig von Faktoren wie Qualität und Aufbau des Materials, ermöglicht, die unabhängig von dem elastischen Teil ausgewählt werden können. Ein weiterer Vorteil der erfindungsgemäß aufgebauten Windel liegt darin, daß die Steifigkeiten nicht nur der genannten dritten Lage, sondern auch der oberen Lage und der rückwärtigen Lage wie auch die Zugfestigkeit des elastischen Teils jeweils in vorbestimmten zugeordneten Bereichen spezifiziert sind, wodurch sichergestellt wird, daß der oben erwähnte Effekt selbst dann erreicht wird, wenn das elastische Teil an der rückwärtigen Lage angeklebt ist. Ein weiterer Vorteil der erfindungsgemäß aufgebauten Wegwerfwindel liegt darin, daß der Wasserdruckwiderstand der dritten Lage ebenfalls in einem vorbestimmten Bereich spezifiziert ist, wodurch sichergestellt wird, daß mögliche Unannehmlichkeiten verhindert werden, die darin bestehen, daß Körperflüssigkeiten, die auf die obere Lage ausgeschieden werden, nach unten auf die Oberfläche der dritten Lage fließen oder zwischen der dritten Lage und der rückwärtigen Lage eindringen, in Kontakt mit der dritten Lage kommen und zu einer Leckage entlang der äußeren Seitenkante dieser Klappe führen könnten.

Im folgenden wird die Erfindung anhand in der Zeichnung dargestellte Ausführungsbeispiele näher erläutert. Es zeigen:
Fig. 1 eine Draufsicht auf eine aufgefaltete Windel mit teilweise weggebrochenen Bereichen, und
Fig. 2A bis 2H unterschiedliche Arten erfindungsgemäßer Anordnungen von wesentlichen Teilen der Windel.

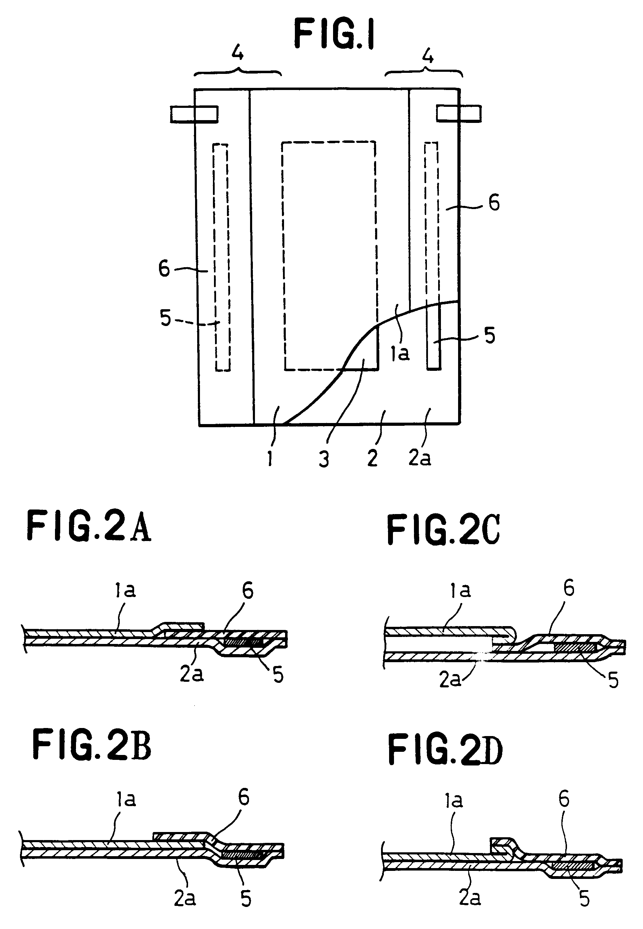

Bezugnehmend auf Fig. 1 weist eine Windel eine flüssigkeitsdurchlässige obere Lage 1, eine flüssigkeitsundurchlässige rückwärtige Lage 2, einen flüssigkeitsabsorbierenden Kern 3, der zwischen diesen beiden Lagen sandwichartig eingelegt ist, und ein Paar Seitenklappen 4 auf, die sich seitlich von einander gegenüberliegenden Seitenrändern des Kerns 3 wegerstrecken.

Jede der Seitenklappen 4 weist einen Bereich 1a der oberen Lage 1 auf, der sich von der zugeordneten äußeren Randkante des Kerns 3 wegerstreckt, einen Bereich 2a der rückwärtigen Lage 2, der sich von der äußeren Seitenkante etwas über den Bereich 1a der oberen Lage hinaus seitlich erstreckt, und eine dritte Lage 6, die an einer Zone des Bereichs 2a der rückwärtigen Lage befestigt ist, in dem die obere Lage nicht vorhanden ist. Zwischen dem Bereich 2a der rückwärtigen Lage und der dritten Lage 6, die gemeinsam einen Teil der Seitenklappe 4 bilden, ist ein sich in Längsrichtung erstreckendes elastisches Teil 5 angeordnet und an der dritten Lage 6 angehaftet. Dieses elastische Teil kann jedoch auch noch an der rückwärtigen Lage 2 angehaftet sein, sodaß es an beiden Lagen befestigt ist.

In den Unterfiguren 2A bis 2H sind verschiedene Arten dargestellt, wie das elastische Teil 5 und die dritte Lage 6 angeordnet sein können.

Fig. 2A zeigt eine Anordnung, bei der der innenliegende Rand der dritten Lage 6 sandwichartig zwischen dem Bereich 1a der oberen Lage und dem Bereich 2a der rückwärtigen Lage eingeklebt ist.

Fig. 2B zeigt eine Anordnung, bei der der innenliegende Rand der dritten Lage 6 an der äußeren Randkante des Bereichs 1a der oberen Lage auf der Oberfläche aufgeklebt ist.

Fig. 2C zeigt eine Anordnung, bei der der innenliegende Rand der dritten Lage 6 nicht nur auf dem Bereich 2a der rückwärtigen Lage, sondern ebenfalls auf dem Bereich 1a der oberen Lage aufgeklebt ist, und zwar auf der Außenrandkante des Bereichs 1a, die nach unten und nach innen gefaltet worden ist, so daß der Innenrand der dritten Lage 6 zwischen diesem umgefalteten Rand und der rückwärtigen Lage zu liegen kommt.

Fig. 2D zeigt eine Anordnung, bei der der innenliegende Rand der dritten Lage 6 zunächst am Bereich 2a der rückwärtigen Lage 2 und dann dauf den Bereich 1a der oberen Lage entlang der Außenrandkante dieser Lage angeklebt ist, wobei diese Außenrandkante nach außen und rückwärts gefaltet worden ist, so daß der innenliegende Rand der dritten Lage 6 zu oberst liegt.

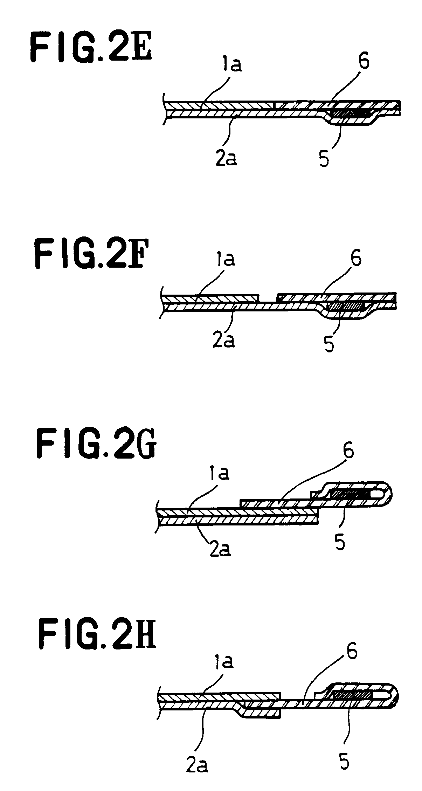

Fig. 2E zeigt eine Anordnung, bei der der innenliegende Rand der dritten Lage 6 auf den Bereich 2a der rückwärtigen Lage aufgeklebt ist und gleichzeitig stumpf an die Außenkante des Bereichs 1a der oberen Lage anstößt.

Bei der in Fig. 2F dargestellten Anordnung ist die innenliegende Randkante der dritten Lage 6 wiederum an dem Bereich 2a der rückwärtigen Lage angeklebt, es ist jedoch zwischen der freien innenliegenden Randkante der dritten Lage 6 und der Randkante des Bereichs 1a ein Spalt gelassen.

Fig. 2G zeigt eine Anordnung, bei der der innenliegende Rand der dritten Lage 6 auf der Oberfläche des Bereichs 1a der oberen Lage angeklebt ist, der Bereich 2a der rückwärtigen Lage ist in diesem Fall genau so lang wie der Bereich 1a der oberen Lage. Das elastische Teil ist in einem durch Zurückschlagen des freien außenliegenden Rands der dritten Lage entstandenen Faltenraum angeordnet. Das Zurückschlagen erfolgt nach oben, das elastische Teil 5 ist durch die Falte quasi eingewickelt.

Fig. 2H zeigt eine Anordnung, bei der der innenliegende Rand der dritten Lage 6 sandwichartig zwischen den jeweiligen, ebenfalls wieder gleich langen Rändern der Bereiche 1a und 2a eingeklebt ist, wobei hier ebenfalls wieder der frei außenliegende Rand der dritten Lage 6 nach oben und rückwärts umgefaltet ist, so daß in dem entstandenen Faltenraum das elastische Teil 5 quasi eingewickelt ist.

Die obere Lage 1 hat eine einheitliche Struktur, die so ausgelegt ist, daß ausgeschiedenes Körperfluid schnell in Richtung des Kerns 3 geleitet wird und dabei wirksam einen Rückfluß von bereits durch den Kern aufgesaugtem Fluid zur Oberfläche der oberen Lage 1 verhindert.

Eine bevorzugte Ausführungsform eines solchen Aufbaus besteht aus einem Vlies mit einem Grundgewicht, d.h. einem Gewicht pro Flächeneinheit von 25 - 35 g/m², einer Dicke von 1 - 2 mm, bestehend aus einer Oberlage höherer Dichte und einer unteren Lage geringerer Dichte, welche beiden Lagen miteinander schmelzverbunden sind. Komponentfasern solcher Vliese können aus den folgenden Materialgruppen wahlweise kombiniert werden: Polypropylen, Polyethylen, konjugiertes Polyethylen/Polypropylen und konjugiertes Polyester/Polyethylen. Eine derartige zweischichtige obere Lage 1 weist eine Steifigkeit von 90 mm oder höher, gemessen mit dem Clark-Steifigkeitstester gemäß dem Japanischen Industriestandard (P8143) in Längsrichtung der Windel auf.

Die dritte Lage 6 kann wahlweise aus einer Gruppe folgender Materialien gewählt werden:
- Vlies mit schmelzverklebtem Fasern,
- Vlies mit haftverbundenen (span-bonded) Fasern,
   wobei alle diese Vliese aus hydrophobischen Fasern hergestellt und einer Waterproof-Behandlung unterzogen worden sind,
- hydrophobischem Kunststoffilm, und
- einem solchen Film, der mit einer Vielzahl von Öffnungen versehen ist.

Die dritte Lage 6 ist luftdurchlässig und hat einen Wasserdruckwiderstand von 20 mm oder höher, vorzugsweise 30 mm oder höher, wobei eine Wasserdruckfestigkeit von 40 mm oder höher, gemessen mit einem Wasserdruckwiderstandstester gemäß dem Japanischen Industriestandard (L1092) bevorzugt wird. Diese dritte Lage 6 weist ein Basisgewicht von weniger als demjenigen der oberen Lage 1 auf, eine Steifigkeit von 10 - 80 mm, gemessen in Längsrichtung der Windel und eine Steifigkeit von 5 - 80 mm, gemessen in Querrichtung der Windel.

Die rückwärtige Lage 2 besteht aus üblicherweise verwendeten Materialien, wie beispielsweise Polyethylenfilm und besitzt eine Steifigkeit von 60 mm oder weniger, sowohl in Längs- als auch in Querrichtung gemäß dem Japanischen Industriestandard gemessen.

Es wird bevorzugt, die genannte Steifigkeit in Längsrichtung der Windel auf 50 - 100 mm bei einem Querschnitt einzustellen, der die rückwärtige Lage 2 und die dritte Lage 6 aufweist.

Das elastische Teil 5 weist beispielsweise eine Vielzahl von Gummifäden, Urethangummistücke oder Urethanschaumstücke auf und besitzt eine Zugfestigkeit, die auf 70 - 700 g eingestellt ist, damit ein geeigneter Druck um die Schenkel des Trägers ausgeübt wird. Das elastische Teil 5 weist an seiner Außenrandkante einen Abstand von 3 mm oder mehr von der Außenrandkante der oberen Lage 1 auf, welche die oben genannte relativ hohe Steifigkeit besitzt,

Im folgenden wird erläutert, weshalb die Steifigkeit der dritten Lage 6 in Längsrichtung der Windel auf 10 - 80 mm eingestellt wird. Eine prozentuale Kontraktion des elastischen Teils 5 ist signifikant durch diese Längssteifigkeit der Seitenklappe 4 beeinflußt, insbesondere in der dritten Lage 6, welche die Seitenklappe 4 bedeckt. Wenn die Zugfestigkeit des elastischen Teils 5 auf 70 - 700 g in Anbetracht eines geeigneten auf den Schenkel aufzubringenden Drucks eingestellt wird, nimmt die prozentuale Kontraktion des elastischen Teils 5, das zwischen der dritten Lage 6 und der rückwärtigen Lage 2 mit einer Steifigkeit von 60 mm oder weniger optimal zu, wie es durch ein experimentelles Ergebnis eines Beispiels gezeigt wird, das weiter unten beschrieben wird. Damit paßt sich das elastische Teil wirkungsvoll der Bewegung des Schenkels des Trägers an und es wird im wesentlichen kein Spalt zwischen der Region der Seitenklappe 4, in der das elastische Teil 5 aufgenommen ist und dem Bein hervorgerufen, weshalb wirkungsvoll jeglicher Ausfluß aus der Windel verhindert wird.

Wenn die besagte Steifigkeit der dritten Lage 6 höher ist als 80 mm wäre die prozentuale Kontraktion des elastischen Teils 5, das zwischen der dritten Lage 6 und der rückwärtigen Lage 2 sandwichartig eingeklebt ist, genauso gering wie bei einer konventionellen Windel. Wenn diese Steifigkeit geringer als 10 mm ist, erhöht sich selbstverständlich die prozentuale Kontraktion, jedoch würde dieses Lagenmaterial mit dieser geringen Steifigkeit eine signifikant niedrige Festigkeit zeigen und das Basisgewicht dieses Lagenmaterials wäre zu niedrig, um bei der Berührung ein angenehmes Gefühl zu erwecken, wenn dieses Material eine Faserstruktur aufweist.

### BEISPIEL

### 1. Windel, aufgebaut nach dem Stand der Technik als Vergleichsbeispiel.

Die Windel weist auf: eine obere Lage, eine rückwärtige Lage, einen zwischen beiden Lagen sandwichartig eingelegten Kern und elastische, jeweils in Seitenklappen, die aus den genannten Lagen bestehen und sich an einander gegenüberliegenden Seitenrändern des Kerns nach außen erstrecken eingelegte elastischen Teile.

### Details der jeweiligen Windelelemente lauten wie folgt:

### Obere Lage:

Faservlies, bestehend aus einer oberen und einer unteren Faserlage, die miteinander schmelzverklebt sind, die obere Faserlage besitzt ein Basisgewicht von 10 g/m² und besteht aus konjugiertem Polyethylen/Polypropylen, die untere Faserlage weist ein Basisgewicht von 20 g/m² auf und besteht aus konjugiertem Polyester/Polyethylen. Die obere Lage besitzt eine Steifigkeit in Längsrichtung von 140 mm und eine Steifigkeit in Querrichtung von 75 mm, gemessen mit dem Clark-Steifigkeitstester gemäß JIS (P8143).

### Kern:

Flockige Pulpe, die als mattenartiges Teil geformt ist, mit einer Steifigkeit von 10 + 5 g cm, gemessen mit einem Tabertester.

### Rückwärtige Lage:

Polyethylenfilm mit einer Steifigkeit von 50 mm sowohl in Längs- als auch in Querrichtung, gemessen mit dem Clark-Steifigkeitstester.

### Elastisches Teil:

Urethanfilm mit einer Zugfestigkeit von 250 g. Das Teil ist in gestrecktem Zustand befestigt, wobei die Längsstreckung doppelt so groß ist wie der Normalzustand. Die Abmessungen sind 150 mm lang und 20 mm breit.

Es wird darauf hingewiesen, daß das elastische Teil jeweils an der oberen und an der rückwärtigen Lage mittels vier Linien heißschmelzenden Klebers, der auf der Ober- und der Unterseite des Teils aufgebracht ist, angeklebt ist, wobei der innere Seitenrand des elastischen Teils von der Außenseitenkante des Kerns einen Abstand aufweist.

### 2. Erfindungsgemäße Windel

Die Windel weist den Aufbau auf, wie er in bezug auf Fig. 2B beschrieben worden ist.

Als dritte Lage wurde ein haftverbundenes (span bonded) Polypropylenfaservlies verwendet, mit einem Basisgewicht von 20 g/m², einer Längssteifigkeit von 50 mm und einer Quersteifigkeit von 40 mm, gemessen mit dem genannten Clark-Steifigkeitstester. Die übrigen Details entsprechen denjenigen unter (1).

### 3. Experimentelle Resultate der prozentualen Kontraktion des elastischen Teils.

Die Sampels wurden jeweils im gedehnten und im nicht gedehnten Zustand gemessen. Dabei wurden die einander gegenüberliegende Enden der Seitenklappen, in denen jeweiligen elastischen Teile, die in den Windeln entsprechend (1) und (2) angeordnet sind, manuell ergriffen und gedehnt, bis die Falten, die sich an den entsprechenden Seitenklappen ausgebildet hatten, verschwunden sind. Die Längen jedes elastischen Teils wurde gemessen, nachdem der Halt an den sich gegenüberliegenden Enden der Seitenklappen gelöst und das elastische Teil sich aus dem gedehnten Zustand wieder zusammengezogen hat (gedehnter Zustand das Doppelte der Originallänge von 150 mm). Dann wurde das Verhältnis der Länge in dem zusammengezogenen Zustand jedes elastischen Teils zur gedehnten Länge 150 mm) bestimmt. Die Windel gemäß (1) zeigte eine prozentuale Kontraktion von 40%, die Windel gemäß (2) zeigte eine prozentuale Kontraktion von 60%.

## Patentansprüche

1. Wegwerfwindel mit einer flüssigkeitsdurchlässigen oberen Lage (1), einer flüssigkeitsundurchlässigen rückwärtigen Lage (2), einem flüssigkeitsabsorbierenden Kern (4), der zwischen diesen beiden Lagen sandwichartig eingelegt ist und zwei Seitenklappen, die sich an einander gegenüberliegenden Rändern des Kerns seitlich nach außen erstrecken, und an denen elastische Teile (5) befestigt sind, wobei jede der Seitenklappen außerhalb der oberen Lage angeordnet ist und eine dritte Lage (6) aufweist, die zumindest einen Teil der genannten Seitenklappen bildet, dadurch **gekennzeichnet**, daß die dritte Lage (6) eine Steifigkeit in Längsrichtung der Windel besitzt, die geringer ist als diejenige der oberen Lage (1).

2. Wegwerfwindel nach Anspruch 1, dadurch **gekennzeichnet**, daß die obere Lage eine Steifigkeit von 90 mm oder höher besitzt, daß die rückwärtige Lage (2) eine Steifigkeit geringer als 60 mm besitzt und daß die dritte Lage (6) eine Steifigkeit von 10 - 80 mm besitzt, wobei das elastische Teil eine Zugfestigkeit aufweist von 70 - 700 g.

3. Wegwerfwindel nach Anspruch 1 oder 2, dadurch **gekennzeichnet**, daß die dritte Lage (6) einen Wasserdruckwiderstand von 20 mm oder höher besitzt und luftdurchlässig ist.

## Claims

1. A disposable nappy with a upper layer (1) permeable to liquid, a rear layer (2) impermeable to liquid, a liquid absorbing core (3) which is disposed in sandwich fashion between these two layers, and two side flaps which extend outwardly at mutually opposite edges of the core and to which elastic parts (5) are fixed, wherein each of the side flaps is arranged outside the upper layer and comprises a third layer (6) which forms at least a part of the said side flaps, characterized in that the third layer (6) has a stiffness in the longitudinal direction of the nappy which is smaller than that of the upper layer (1).

2. A disposable nappy according to claim 1, characterized in that the upper layer has a stiffness of 90 mm or more, in that the rear layer (2) has a stiffness less than 60 mm and in that the third layer (6) has a stiffness of 10 - 80 mm, while the elastic part has a tensile strength of 70 - 700 g.

3. A disposable nappy according to claim 1 or 2, characterized in that the third layer (6) has a resistance to water pressure of 20 mm or more and is translucent.

## Revendications

1. Couche-culotte jetable comprenant une couche supérieure (1) perméable aux liquides, une couche arrière (2) imperméable aux liquides, un corps central (3) absorbant les liquides, emprisonné en sandwich entre ces deux couches, et deux rabats latéraux, qui s'étendent latéralement vers l'extérieur sur des bords mutuellement opposés du corps central, et auxquels sont attachés des éléments élastiques (5), chacun des deux rabats latéraux étant disposé à l'extérieur de la couche supérieure et présentant une troisième couche (6), qui forme au moins une partie des rabats latéraux précités, caractérisée en ce que la troisième couche (6) a une rigidité dans le sens longitudinal de la couche-culotte, qui est inférieure à celle de la couche supérieure (1).

2. Couche-culotte jetable suivant la revendication 1, caractérisée en en ce que la couche supérieure a une rigidité de 90 mm ou plus, et en ce que la couche arrière (2) a une rigidité de moins de 60 mm et en ce que la troisième couche (6) a une rigidité de 10 - 80 mm, l'élément élastique présentant une résistance à la traction de 70 - 700 g.

3. Couche-culotte jetable suivant l'une des revendications 1 ou 2, caractérisée en ce que la troisième couche (6) présente une résistance à la pression de l'eau de 20 mm ou plus, et est perméable à l'air.
